Europäisches Patentamt

European Patent Office

Office européen des brevets

(19)

(11) **EP 0 592 632 B1**

(12) **FASCICULE DE BREVET EUROPEEN**

(45) Date de publication et mention
de la délivrance du brevet:
**04.08.1999 Bulletin 1999/31**

(21) Numéro de dépôt: **93907917.4**

(22) Date de dépôt: **31.03.1993**

(51) Int Cl.[6]: **G01N 21/77**, G01N 21/78,
G01N 21/31

(86) Numéro de dépôt international:
**PCT/FR93/00325**

(87) Numéro de publication internationale:
**WO 93/20431 (14.10.1993 Gazette 1993/25)**

(54) **PROCEDE POUR LA MESURE DE LA CONCENTRATION DE GAZ CARBONIQUE DISSOUS DANS UNE EAU DE MER ET DISPOSITIF POUR SA MISE EN UVRE**

VERFAHREN ZUR MESSUNG DES INHALTS VON IN SEEWASSER AUFGELÖSTEM KOHLENSTOFFDIOXID, UND VORRICHTUNG ZUR AUSFÜHRUNG DESSELBEN

METHOD FOR MEASURING THE CONCENTRATION OF CARBON DIOXIDE DISSOLVED IN SEA WATER, AND DEVICE THEREFOR

(84) Etats contractants désignés:
**AT BE CH DE DK ES FR GB GR IE IT LI LU MC NL PT SE**

(30) Priorité: **03.04.1992 FR 9204120**

(43) Date de publication de la demande:
**20.04.1994 Bulletin 1994/16**

(73) Titulaire: **CENTRE NATIONAL DE LA RECHERCHE SCIENTIFIQUE
75007 Paris (FR)**

(72) Inventeurs:
 • **MERLIVAT, Liliane
   F-75005 Paris (FR)**
 • **MICHARD, Gil
   F-75015 Paris (FR)**

 • **CIABRINI, Jean-Pierre
   F-94450 Limeil-Brevannes (FR)**
 • **DU CHAFFAUT, Maurice
   F-75013 Paris (FR)**
 • **LEFEVRE, Nathalie
   F-94420 Le Plessis-Trévis (FR)**
 • **BRIENT, Bernard
   F-75012 Paris (FR)**
 • **DANGUY, Théodore
   F-75013 Paris (FR)**

(74) Mandataire: **Phélip, Bruno et al
   c/o Cabinet Harlé & Phélip
   7, rue de Madrid
   75008 Paris (FR)**

(56) Documents cités:
   **EP-A- 0 046 601        DE-A- 3 641 645
   US-A- 3 902 812         US-A- 5 039 492**

## Description

La présente invention est relative à un procédé pour la mesure de la concentration du gaz carbonique dissous dans une eau de mer et à un dispositif pour sa mise en oeuvre.

Par ses propriétés radiatives, le gaz carbonique ($CO_2$) présent dans l'atmosphère joue un rôle important pour déterminer les caractéristiques de notre climat puisqu'il est responsable pour environ un tiers de l'effet de serre naturel autour de la terre.

Le développement des activités humaines a entraîné une augmentation de 25% de la concentration atmosphérique de $CO_2$ depuis le début de la révolution industrielle induisant un effet de serre complémentaire et par suite une perturbation du système climatique. On est maintenant confronté au problème de pouvoir prédire l'évolution de la concentration du gaz dans l'atmosphère au cours des années à venir en réponse aux développements des activités de l'homme (utilisation de combustibles fossiles, déforestation.....).

L'océan joue un rôle prépondérant dans le contrôle de la concentration du $CO_2$ dans l'atmosphère puisqu'il représente le puits majeur pour absorber le gaz, la taille du réservoir océanique étant 60 fois supérieure à celle du réservoir atmosphérique. Toutefois, la vitesse avec laquelle le gaz pénètre dans l'océan n'est que très mal connue, un facteur 3 existant entre les valeurs extrêmes des estimations du flux de $CO_2$ absorbé par l'océan mondial. Ce flux est compris entre $0.8.10^{15}$ et $2.4.10^{15}$ grammes de carbone par an.

Un des paramètres nécessaires pour calculer la valeur de ce flux est la concentration du gaz dissous à la surface de l'océan global. Il s'agit d'une quantité présentant une très grande variabilité spatiale et temporelle, les processus à l'origine de cette variabilité étant d'ordre physique, chimique et biologique.

La connaissance actuelle de la distribution de la concentration de $CO_2$ à l'échelle de l'océan global repose sur des séries de données acquises lors de campagnes océanographiques dédiées. Cet échantillonnage est très incomplet, des régions entières de l'océan telles que le Pacifique Sud ou l'Antarctique n'étant absolument pas documentées dans le jeu de données actuelles.

Pour pallier à ces insuffisances, on cherche à pouvoir disposer de systèmes autonomes captant l'information à la surface de l'océan et la retransmettant, par exemple par voie hertzienne à partir de bouées instrumentées.

A ce jour néanmoins, il n'existe pas de capteur permettant la mesure de la concentration de $CO_2$ dissous dans l'eau de mer et suffisamment autonome pour être implanté sur une boue dérivante. A l'heure actuelle en effet, les mesures sont faites à partir de bateaux qui mettent en oeuvre les techniques par infra-rouge ou chromatographie en phase gazeuse, qui nécessitent en particulier l'utilisation de bouteilles de gaz étalon comprimé. L'utilisation de ces techniques sur des bouées autonomes est donc inenvisageable.

La présente invention a pour but de pallier ces différents inconvénients. Elle propose notamment un dispositif autonome pour la mesure de $CO_2$ dans de l'eau de mer.

La présente invention a donc pour objet un procédé de mesure de la teneur en $CO_2$ dissous dans une eau de mer, dans lequel:

- on plonge une cellule de mesure (3) dans l'eau de mer, cette cellule de mesure (3) comportant une solution à indicateur coloré sur laquelle les mesures de pH par colorimétrie peuvent être effectuées et dont la concentration en $CO_2$ s'équilibre avec celle de l'eau de mer;
- on réalise, sur ladite solution, une mesure du pH par colorimétrie à l'aide d'un spectromètre;

caractérisé en ce que la mesure du pH comprend trois mesures de l'absorbance optique A de la solution dont

- la première est effectuée à une longueur d'onde ($\lambda_M$) caractéristique d'un maximum d'absorption de la forme basique du colorant;
- la deuxième est effectuée à une longueur d'onde ($\lambda_B$) à laquelle le colorant n'absorbe pas; et
- la troisième est effectuée à une longueur d'onde ($\lambda_I$) vers le point isobestique du colorant afin de tenir compte de la stabilité limitée du colorant dans le temps;

ainsi qu'un étalonnage pour déterminer un coefficient $A_M$ caractéristique de la cellule de mesure (3) et du colorant utilisé, et un coefficient $K_M$ prenant en compte les caractéristiques d'émission et de détection du spectromètre; et
en ce que l'on déduit des résultats des trois mesures la valeur pH représentant de la teneur en $CO_2$ selon la relation $P_H = P_K + \log A/(A_M-A)$, $P_K$ étant une constante caractéristique de l'indicateur coloré contenu dans la cellule de mesure (3) et l'absorbance A étant obtenue par la relation $A = \log I_{eB}/I_{eM} + K_M$ avec $I_{eB}$ comme intensités lumineuses mesurées à la sortie de la cellule de mesure (3) aux longueurs d'onde correspondantes ($\lambda_B$, $\lambda_M$).

Avantageusement, l'indicateur coloré est le bleu de Thymol, la première mesure étant réalisée à une longueur d'onde caractéristique du maximum d'absorption de la forme basique, soit environ 596 nanomètres, la longueur d'onde isobestique étant de 493 nanomètres, la longueur d'onde à laquelle le colorant n'absorbe pas étant choisie au-delà de 700 nanomètres.

L'invention a encore pour objet un dispositif pour la mesure de la teneur en $CO_2$ dissous dans l'eau de mer comprenant spectromètre muni d'une source émettrice notamment aux longueurs d'onde de mesure, et d'un récepteur, ledit dispositif comportant également une cellule de mesure comportant la solution à indicateur coloré, ladite cellule de mesure étant destinée à être plongée dans l'eau de mer et étant d'une structure lui permettant d'équilibrer sa concentration de $CO_2$ dissous avec celle de l'eau de mer, des moyens pour conduire le faisceau lumineux de la source émettrice jusqu'à une entrée optique de ladite cellule, puis d'une sortie optique de ladite cellule jusqu'au récepteur, caractérisé en ce qu'il comprend en outre des moyens pour filtrer successivement le faisceau optique en sortie de la cellule aux différentes longueurs d'onde nécessaires pour réaliser les mesures selon le procédé précité.

Le dispositif est avantageusement complété par les différentes caractéristiques suivantes seules ou selon toutes leurs combinaisons techniquement possibles:

- le spectromètre comporte un séparateur optique placé entre la cellule de mesure et l'optique d'entrée de la cellule de mesure et un photorécepteur de référence et une lentille de voie de référence, le séparateur optique prélevant une partie du flux émis par la source et la lentille de voie de référence l'adressant sur le photorécepteur de référence, la mesure fournie par le dispositif résultant de la comparaison entre le signal fourni par le photorécepteur de référence et celui fourni par le récepteur.
- les moyens de conduction optique sont des fibres optiques;
- la cellule comprend un logement recevant une solution d'indicateur coloré, ce logement étant fermé sur au moins une de ses parois par une membrane semi-perméable au $CO_2$ dissous;
- l'évidement de la cellule est une rainure parallélépipédique traversant le boîtier celle-ci et fermée sur quatre de ses côtés par la matière constituant ledit boîtier et sur deux autres de ses côtés par des membranes semi-perméables;
- ces membranes sont maintenues sur ledit boîtier par l'intermédiaire de pièces de maintien portant sur des joints interposés entre lesdites pièces de maintien et lesdites membranes;
- les moyens pour véhiculer l'impulsion lumineuse sont des fibres optiques dont les extrémités débouchent dans ladite rainure au niveau des bords d'extrémités longitudinales de celle-ci;
- trois filtres interférentiels sont disposés sur un plateau tournant interposé entre d'une part l'extrémité de la fibre optique véhiculant l'impulsion lumineuse en sortie de la cellule et d'autre part le détecteur du spectromètre, lesdits trois filtres passant successivement au cours de la rotation dudit plateau tournant au droit de l'extrémité de ladite fibre et du détecteur qui lui est en regard.

La description qui suit est purement illustrative et non limitative. Elle doit être lue en regard des dessins annexés sur lesquels:

La Figure 1 est une représentation synoptique d'un dispositif conforme à un mode de réalisation particulier de l'invention.

La Figure 2 est une vue en perspective éclatée du capteur optique du dispositif de la Figure 1;

La Figure 3 est une vue en coupe axiale du capteur de la Figure 2;

La Figure 4 est une représentation schématique des spectres d'absorbance en fonction de la longueur d'ondes donnée en nm, d'une part des formes acide et basique de l'indicateur coloré choisi pour les capteurs du dispositif, et d'autre part d'une de ses formes intermédiaires;

La Figure 5 est une représentation synoptique des éléments du spectromètre du dispositif de la Figure 1;

La Figure 6, enfin, est une représentation graphique d'une courbe de concentration de $CO_2$ dissous à la surface de l'eau de mer en fonction de la grandeur de mesure du dispositif.

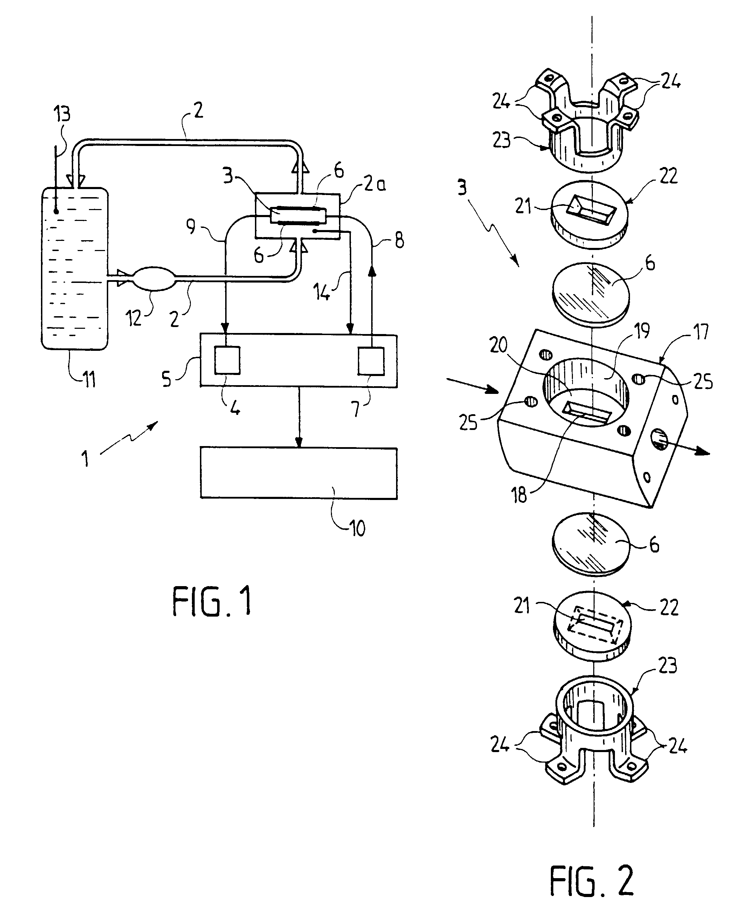

On voit sur la Figure 1 que le dispositif 1 conforme à un mode de réalisation particulier de l'invention qui y a été représenté, comprend un réservoir d'eau de mer 11 et un circuit 2 de tuyauterie dans lequel l'eau de mer circule. Ce circuit 2 envoie de l'eau de mer pompée par l'intermédiaire d'une pompe 12, dudit réservoir 11 sur une enceinte de mesure 2a, puis renvoie l'eau de mer qui a traversé ladite enceinte 2a dans ledit réservoir 11. Des capteurs de températures 13 et 14 sont disposés respectivement au niveau dudit réservoir 11 et de ladite enceinte de mesure 2a. Une cellule de mesure 3 est disposée dans ladite enceinte 2a.

La mesure de la concentration de $CO_2$ dissous à la surface de l'eau de mer est réalisée par une mesure par colorimétrie du pH de la cellule 3. La cellule 3 est munie à cet effet d'une solution contenant un indicateur coloré.

Le principe permettant de passer d'une mesure de $P_H$ à une mesure de concentration de $CO_2$ dissous est le suivant. La distribution des composés du carbone dissous dans l'eau de mer est régi par des équilibres chimiques dont les constantes sont connues ainsi que leurs variations avec la température. L'application de ces lois d'équilibre montre que la valeur de la pression partielle de $CO_2$ ($PCO_2$) et le pH d'une solution d'alcalinité A connue et maintenue constante sont liés par la relation:

$$(1) \qquad PCO_2 = \frac{A}{S \cdot \dfrac{K_1}{(H^+)} + \dfrac{2\,K_1 . K_2}{(H^+)^2}}$$

où:

- $K_1$ et $K_2$ sont les valeurs de la première et deuxième constante d'acidité de l'acide carbonique dans l'eau de mer.
- S, la solubilité de $CO_2$ dans l'eau de mer

avec

$$P_H = - \log H^+$$

Le principe de la mesure de pH et le choix de l'indicateur coloré seront décrits plus loin plus en détails.

Sur le schéma synoptique représenté sur la Figure 1, on voit que la cellule 3 contenant l'indicateur coloré pour la mesure du pH est muni de membranes 6 sur deux de ses faces, ces membranes étant semi-perméables au $CO_2$ dissous. La cellule 3 baigne dans l'eau de mer que lui fournit la pompe 12, de sorte que le $CO_2$ dissous dans la solution de la cellule 3 contenant l'indicateur sera en équilibre avec le $CO_2$ dissous dans l'eau de mer. Les moyens de mesure de pH par colorimétrie comportent principalement quant à eux un spectromètre S muni d'un capteur 4 et d'une source émettrice 7 envoyant sur la cellule 3 un faisceau lumineux, par l'intermédiaire d'une fibre optique 8. Ce faisceau, après avoir traversé la cellule 3, sera renvoyé dans une fibre optique de sortie 9 sur le récepteur 4 lui-même relié à une électronique d'enregistrement 10.

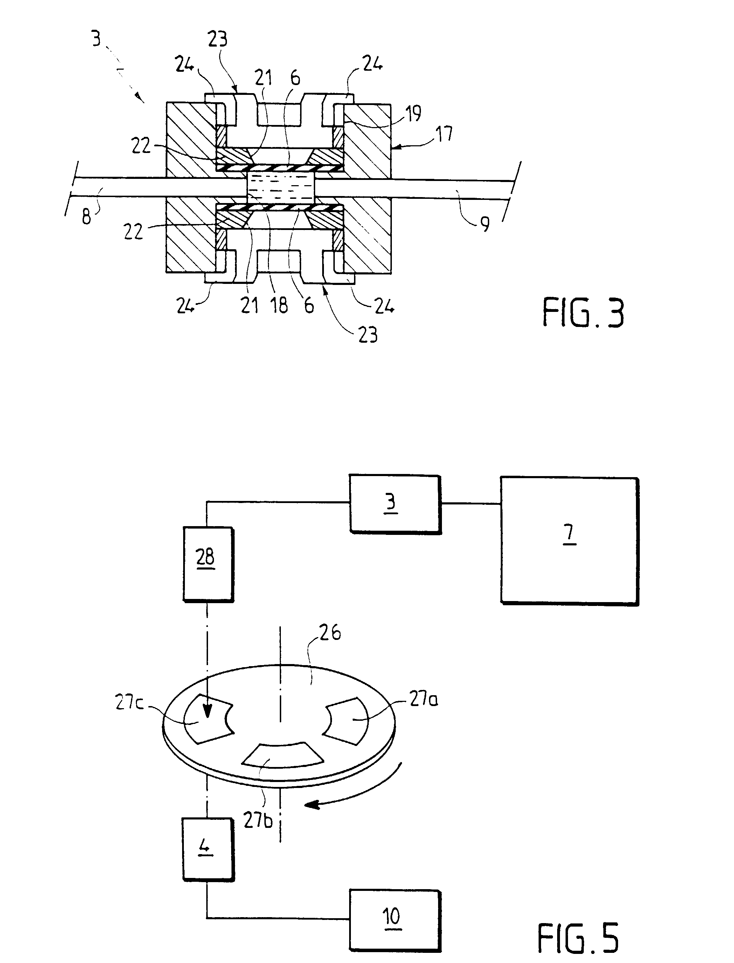

On se réfère plus particulièrement aux Figures 2 et 3 sur lesquelles a été représentée la cellule de mesure 3. Ainsi qu'on peut le voir sur ces figures, cette cellule 3 comprend principalement un boîtier 17 traversé en sa partie centrale par une fente 18 dans laquelle est destinée à être disposée la solution contenant l'indicateur coloré. De part et d'autre de cette fente 18, le boîtier 17 comprend des évidements sensiblement cylindriques 19 et au niveau du fond 20 desquels débouche la fente 18. Ces évidements 19 sont destinés à recevoir d'une part les membranes 6 semi-perméables au $CO_2$ dissous et d'autre part des joints 22 ainsi que des éléments de fixation 23. De telles membranes 6 sont appuyées sur le fond 20 par un tel joint 22. Les joints 22 sont munis de rainures 21 pour le passage de l'eau de mer jusqu'aux membranes 6. Ces rainures 21 sont de section trapézoïdales s'évasant à partir des membranes 6 sur lesquelles ils sont disposés vers l'extérieur de la cellule et ont une petite base correspondant sensiblement à la largeur de la fente 18 dans laquelle est disposée la solution contenant l'indicateur coloré. Les éléments de maintien 23 sont quant à eux des couronnes cylindriques dont les parois extérieures sont destinées à épouser les parois intérieures des évidements 19 et qui sont munies de pattes radiales 24 destinées à venir en appui sur les parois extérieures du boîtier 17 autour de l'évidement 19. Ces pattes 24 sont destinées à être traversées chacune par le corps d'une vis (non représentée) dont le filetage coopère avec un filetage dont sont munis des alésages 25 traversant le boîtier 17. Les axes des alésages 25 sont parallèles aux axes des évidements 19. La tête des vis précitées vient en appui sur les pattes 24.

Les membranes semi-perméables 6 sont par exemple des membranes en silicone fabriquées en laboratoire à partir d'une solution de polymère commercialisée par la société DOW CORNING sous le N° 92009. Les joints 22 peuvent être des rondelles en laiton ou en bronze. Le volume de la fente 19 est de 14 microlitres. Cette fente a une longueur de 10 mm et une largeur et une épaisseur de 1,2 mm chacune. Ces dimensions ont été optimisées pour que les surfaces d'échanges en $CO_2$ dissous soient importantes. A chacune de ses extrémités longitudinales, la fente 18 reçoit l'extrémité d'une des monofibres 8 ou 9. Ces monofibres 8 ou 9 sont en silice et sont de 1 mm de diamètre. Ces fibres 8 ou 9 sont maintenues sur le boîtier 17 par des brides de maintien qui n'ont pas été représentées ici. Des rondelles sont également prévues pour assurer l'étanchéité entre lesdites fibres et ledit boîtier au niveau de la fente 18.

La mesure du pH par colorimétrie se réalise à partir de la détermination de la fraction x de l'indicateur coloré qui est sous forme basique, avec

$$P_H = P_K + \log x/(l-x) \qquad (2)$$

où $P_K$ est une constante caractéristique de l'indicateur contenu dans la cellule 3 et la fente 18.

L'indicateur coloré choisi est le bleu de Thymol, qui est une sulfonephtaleine $C_{27}H_{30}O_5S$. Les propriétés d'un tel indicateur pour la mesure colorimétrique d'un pH dans un milieu proche de l'eau de mer ont déjà fait l'objet d'une étude dont les résultats ont notamment été publiés dans un article de Jean-Pierre CIABRINI, LEFEVRE et Gil MICHARD - C.R. Académie de Science de Paris, Tome 313, Série II, pages 629-633, 1991 - Chimie Analytique - Etude d'un indicateur permettant la mesure colorimétrique du pH et de la pression partielle du dioxyde de carbone de l'eau de mer: la thymolsulfonephtaléine. Un tel indicateur est très sensible aux variations de pH. La valeur de son $P_K$ est voisine de celle du pH de l'eau de mer et donc adaptée au problème que cherche à résoudre l'invention.

L'indicateur coloré est introduit dans une solution reproduisant synthétiquement l'eau de mer, sans son aspect biologique. Ce solvant marin est d'une salinité de 35 pour 1000 et est réalisé à partir de produits commercialisés par la société MERCK avec la composition suivante pour 1 kg de solution:

204,5 ml de NaCl, 2 M
10 ml de KCl, 1 M
64 ml de $MgCl_2$, 1 M
28 ml de $Na_2SO_4$, 1 M
21 ml de $NaHCO_3$ 0,1 M

Dans ces proportions de solution il est ajouté 20 ml de bleu de Thymol à $2.10^{-3}$ M. La concentration

du colorant dans la solution est donc de $4.10^{-5}$ M/kg.

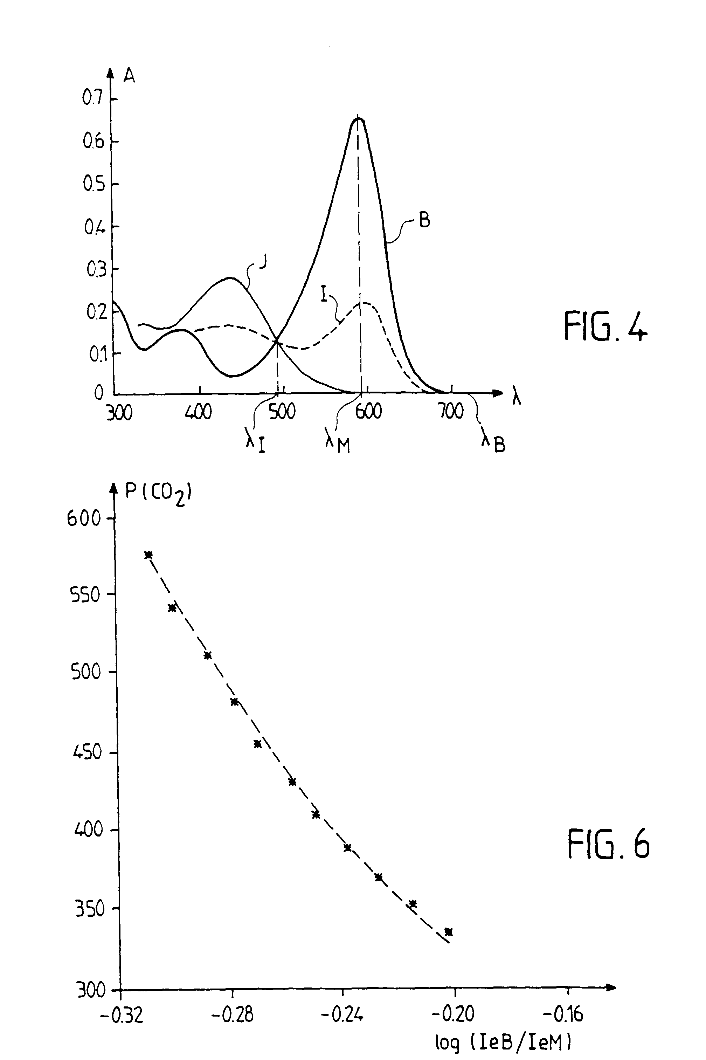

Sur la Figure 4, ont été représentés les spectres B, J et I des formes respectivement basique (forme B bleu), acide (forme J jaune) et intermédiaire (forme I). Les courbes J et B se croisent en un point appelé point isobestique dans lequel passe également par conséquent toute courbe intermédiaire I.

Dans l'équation (2) la mesure de x se ramène à une mesure d'absorption optique par application de la loi de Beer Lambert:

$$A = E_M.c.l.x \qquad (3)$$

où:

A représente l'absorbance optique de la solution à la longueur d'onde $\lambda_M$ caractéristiques du maximum d'absorption de la forme basique (bleu) du colorant $\lambda_M$ est égal à 596 nm.

- $E_M$ est le coefficient d'extinction de la forme basique du colorant à la longueur d'onde $\lambda_M$.
- c est la concentration du colorant. Elle est égale à $4.10^{-5}$ M.kg.
- l est la longueur du trajet optique dans la cellule de mesure.

La formule (3) prend en compte le fait que l'absorbance de la forme acide est négligeable à $\lambda_M$, ce qui a été vérifié expérimentalement.

On introduit $A_M$ l'absorbance du colorant lorsqu'il est entièrement sous forme basique.
D'après (3) on a:

$$A_M = E_M.c.l. \qquad (4)$$

$A_M$ est un paramètre caractéristique de la cellule de mesure et du colorant utilisé.
La combinaison des relations (2), (3) et (4) permet d'écrire:

$$P_H = P_K + \log A/(A_M - A) \qquad (5)$$

La détermination expérimentale de A repose sur la mesure des intensités lumineuses $li_M$ et $le_M$ par la relation

$$A = \log li_M/le_M \qquad (6)$$

où:

- $le_M$ est l'intensité lumineuse mesuré en sortie de la cellule de mesure.
- $li_M$ représente l'intensité lumineuse qui serait obtenue en sortie de cellule si la solution colorée était remplacée par une solution incolore.

Le dispositif expérimental ne permet pas de faire directement la mesure de $li_M$. Cette difficulté a été pallié en faisant une mesure complémentaire à une longueur d'onde $\lambda_B$ à laquelle le colorant n'absorbe pas. On a donc:

$$li_M = le_B \qquad (7)$$

$\lambda_B$ est choisie égal à 730 nm.

Les relations (6) et (7) conduisent à:

$$A = \log le_B/le_M + K_M \qquad (8)$$

où

- $le_B$ et $le_M$ sont les deux intensités lumineuses mesurées à la sortie de la cellule à 730 nm et 596 nm.
- $K_M$ est un facteur d'appareil qui prend en compte en particulier la courbe d'émission spectrale de la lampe du spectromètre ainsi que les caractéristiques des détecteurs. $K_M$ est déterminé par étalonnage.

Une mesure complémentaire d'absorbance $A_I$ est faite à la longueur d'onde du point isobestique du colorant, $\lambda_I$. A cette longueur d'onde le coefficient d'extinction, $E_I$, est égal pour les formes acide et basique du colorant. La motivation pour faire cette mesure tient à ce que la stabilité du colorant dans le temps est limitée. La valeur de c dans l'expression de A va décroître (3). Cette décroissance peut être corrigée par une mesure à la longueur d'onde du point isobestique. En effet, d'après (3), on a:

$$A_I/A_M = E_I/E_M \qquad (9)$$

D'autre part en appliquant la relation (8) pour les mesures faites à la longueur d'onde $\lambda_I$, on a:

$$A_I = \log le_B/le_I + K_I \qquad (10)$$

où $le_I$ est l'intensité lumineuse mesurée à la sortie de la cellule d'onde $\lambda_I$.

En résumé, pour tenir compte des évolutions au vieillissement de la lampe de la source émettrice et du bleu de Thymol, il est réalisé pour la détermination de la concentration de $CO_2$ dissous des mesures d'intensité lumineuse aux trois longueurs d'ondes $\lambda B$, $\lambda M$, et $\lambda I$.

Les coefficients d'étalonnage $A_M$ et $K_M$ auront préalablement été déterminés à l'aide de solutions ayant des teneurs de $PCO_2$ connu et donc de pH connu.

Les coefficients $A_I$ et $K_I$ sont quant à eux contrôlés lors des mesures réalisées par le dispositif. Notamment, la constance du rapport $A_I/A_M$ constitue un critère permettant d'évaluer la stabilité du colorant dans le temps.

Le spectromètre 5 du dispositif a été plus particulièrement représenté sur la Figure 5. Il comprend principalement une source 7 qui envoie par l'intermédiaire d'une fibre optique 8 un faisceau lumineux sur la cellule 3. Une fibre 9 en sortie de cette cellule 8 renvoie quant à elle le faisceau après passage dans ladite cellule 3 sur un plateau 26 tournant 26 muni de trois filtres interférentiels 27a, 27b, 27C répartis à 90° les uns des autres et filtrant le faisceau lumineux de sortie aux longueurs d'onde de mesure indiquées précédemment (493 nanomètres, 596 nanomètres et 730 nanomètres). Une quatrième position sur le plateau est une position noire réservée au contrôle de l'électronique du spectromètre. Au droit de l'élément 28 du spectromètre maintenant l'extrémité libre de la fibre 9, est disposé le détecteur 4 des faisceaux lumineux ayant passé à travers le filtre 27a, 27b ou 27c. Ce récepteur 4, qui est du type à photodiode, est lui-même relié à l'électronique 10 destinée à amplifier et digitaliser la mesure relevée.

L'entraînement du plateau 26 peut être réalisé par un moteur pas à pas, le plateau s'arrêtant devant chaque filtre 27a, 27b ou 27C. Egalement, le plateau 26 peut être entraîné de façon continue en rotation sur lui-même. A chaque cycle de mesure, le plateau 26 va, à partir d'une position de repos où la position noire est par exemple en regard du détecteur 4, et effectuer un tour complet. Le passage d'un des trois filtres 27a à c dans le faisceau lumineux se traduit en sortie du détecteur 4 par une impulsion dont la forme dépend de la largeur relative du filtre et du faisceau lumineux et dont la largeur est fonction de la vitesse de rotation du plateau. Si l'axe de rotation du plateau possède un jeu suffisamment faible, le maximum de l'impulsion est, à intensité lumineuse constante, répétitif. La mesure est donc effectué au maximum de chaque signal.

Le spectromètre peut avantageusement comporter un séparateur optique placé entre la cellule de mesure 3 et l'optique d'entrée de la cellule de mesure 3 et un photorécepteur de référence et une lentille de voie de référence, le séparateur optique prélevant une partie du flux émis par la source 7 et la lentille de voie de référence l'adressant sur le photorécepteur de référence, la mesure fournie par le dispositif résultant de la comparaison entre le signal fourni par le photorécepteur de référence et celui fourni par le récepteur.

Le spectromètre 5 peut également être réalisé en optique traditionnelle, sans fibre. Des lentilles ou optiques assurent alors la conjugaison de la source 7 avec le récepteur photoélectrique 4. Le filtre et la cellule de mesure 3 sont traversés en faisceau approximativement parallèle.

L'étalonnage du capteur est quant à lui effectué en laboratoire par bullage d'un gaz de teneur en $CO_2$ connu suivi d'ajout d'acide en quantité également connue. Le domaine de la teneur en $CO_2$ étudié est compris entre 330 microatmosphères et 600 microatmosphères qui sont des valeurs représentatives des concentrations existant à la surface de l'océan. A titre d'exemple, on a représenté sur la Figure 7, la courbe d'étalonnage obtenue avec le capteur conforme à l'invention. La pression de $CO_2$ y est donnée en microatmosphère, c'est-à-dire environ en $10^{-1}$Pa. La grandeur en abscisse est $\log(\mathrm{le}_B/\mathrm{le}_M)$. Ce capteur a une sensibilité de l'ordre de 1 microatmosphère. La distribution des points expérimentaux autour de la courbe d'étalonnage est de 1%.

Les signes de référence insérés après les caractéristiques techniques mentionnées dans les revendications, ont pour seul but de faciliter la compréhension de ces dernières, et n'en limitent aucunement la portée.

**Revendications**

1. Procédé de mesure de la teneur en $CO_2$ dissous dans une eau de mer, dans lequel:

   - on plonge une cellule de mesure (3) dans l'eau de mer, cette cellule de mesure (3) comportant une solution à indicateur coloré sur laquelle les mesures de pH par colorimétrie peuvent être effectuées et dont la concentration en $CO_2$ s'équilibre avec celle de l'eau de mer;
   - on réalise, sur ladite solution, une mesure du pH par colorimétrie à l'aide d'un spectromètre;

   caractérisé en ce que la mesure du pH comprend trois mesures de l'absorbance optique A de la solution dont

   - la première est effectuée à une longueur d'onde ($\lambda_M$) caractéristique d'un maximum d'absorption de la forme basique du colorant;
   - la deuxième est effectuée à une longueur d'onde ($\lambda_B$) à laquelle le colorant n'absorbe pas; et
   - la troisième est effectuée à une longueur d'onde ($\lambda_I$) vers le point isobestique du colorant afin de tenir compte de la stabilité limitée du colorant dans le temps;

   ainsi qu'un étalonnage pour déterminer un coefficient $A_M$ caractéristique de la cellule de mesure (3) et du colorant utilisé, et un coefficient $K_M$ prenant en compte les caractéristiques d'émission et de détection du spectromètre; et
   en ce que l'on déduit des résultats des trois mesures la valeur pH représentant de la teneur en $CO_2$ selon la relation $P_H = P_K + \log A/(A_M - A)$, $P_K$ étant une constante caractéristique de l'indicateur coloré contenu dans la cellule de mesure (3) et l'absorbance A étant obtenue par la relation $A=\log I_{eB}/I_{eM} + K_M$ avec $I_{eB}$ comme intensités lumineuses mesurées à la sortie de la cellule de mesure (3) aux longueurs d'onde correspondantes ($\lambda_B$, $\lambda_M$).

2. Procédé selon la revendication 1, caractérisé en ce que l'indicateur coloré est le bleu de Thymol, la première mesure étant réalisée à une longueur d'onde ($\lambda_I$) d'environ 596 nanomètres, la deuxième mesure étant réalisée à une longueur d'onde ($\lambda_I$) d'environ 493 nanomètres, et la troisième mesure étant réalisée à une longueur d'onde ($\lambda_B$) au-delà de 700 nanomètres.

3. Dispositif pour la mesure de la teneur en $CO_2$ dissous dans l'eau de mer comprenant un spectromètre muni d'une source (7) émettrice notamment aux longueurs d'onde de mesure, et d'un récepteur (4), ledit dispositif comportant également une cellule de mesure (3) comportant la solution à indicateur coloré, ladite cellule de mesure (3) étant destinée à être plongée dans l'eau de mer et étant d'une structure lui permettant d'équilibrer sa concentration de $CO_2$ dissous avec celle de l'eau de mer, des moyens (8, 9) pour conduire le faisceau lumineux de la source émettrice (7) jusqu'à une entrée optique de ladite cellule (3), puis d'une sortie optique de ladite cellule (3) jusqu'au récepteur (4), caractérisé en ce qu'il comprend en outre des moyens (26, 27a, 27b, 27c) pour filtrer successivement le faisceau optique en sortie de la cellule (3) aux différentes longueurs d'onde nécessaires pour réaliser les mesures selon le procédé des revendications 1 ou 2.

4. Dispositif selon la revendication 3, caractérisé en ce que le spectromètre comporte un séparateur optique placé entre la cellule de mesure (3) et l'optique d'entrée de la cellule de mesure (3) et un photorécepteur de référence et une lentille de voie de référence, le séparateur optique prélevant une partie du flux émis par la source (7) et la lentille de voie de référence l'adressant sur le photorécepteur de référence, la mesure fournit par le dispositif résultant de la comparaison entre le signal fourni par le photorécepteur de référence et celui fourni par le récepteur.

5. Dispositif selon l'une des revendications 3 ou 4, caractérisé en ce que la cellule (3) comprend un logement (18) recevant une solution d'indicateur coloré, ce logement étant fermé sur au moins une de ses parois par une membrane (6) semi-perméable au $CO_2$ dissous.

6. Dispositif selon la revendication 5, caractérisé en ce que l'évidement (18) de la cellule (3) est une rainure parallélépipédique traversant le boîtier (17) celle-ci et fermée sur quatre de ses côtés par la matière constituant ledit boîtier (17) et sur deux autres de ses côtés par des membranes (6) semi-perméables.

7. Dispositif selon la revendication 6, caractérisé en ce que ces membranes (6) sont maintenues sur ledit boîtier (17) par l'intermédiaire de pièces de maintien (23) portant sur des joints (22) interposés entre lesdites pièces de maintien (23) et lesdites membranes (6).

8. Dispositif selon l'une quelconque des revendications 6 ou 7, caractérisé en ce que les moyens pour véhiculer l'impulsion lumineuse sont des fibres optiques (8, 9) dont les extrémités débouchent dans ladite rainure (18) au niveau des bords d'extrémités longitudinales de celle-ci.

9. Dispositif selon la revendication 8, caractérisé en ce qu'il comprend trois filtres interférentiels (27a, 27b, 27c) disposés sur un plateau tournant (26) interposé entre d'une part l'extrémité de la fibre optique (9) véhiculant l'impulsion lumineuse en sortie de la cellule (3) et d'autre part le détecteur (4) du spectromètre, lesdits trois filtres (27a, 27b, 27c) passant successivement au cours de la rotation dudit plateau tournant (26) au droit de l'extrémité de ladite fibre (9) et du détecteur (4) qui lui est en regard.

10. Dispositif selon l'une quelconque des revendications 3 à 9, caractérisé en ce que les moyens de conduction optique (8, 9) sont des fibres optiques.

**Patentansprüche**

1. Verfahren zur Messung des Gehaltes von in Meerwasser gelöstem $CO_2$, bei dem:

   - eine Meßzelle (3) in das Meerwasser getaucht wird, die eine Farbindikatorlösung enthält, mit der die pH-Messungen durch Kolorimetrie durchgeführt werden können und deren $CO_2$-Konzentration sich derjenigen des Meerwassers angleicht;

   - die Lösung einer pH-Messung durch Kolorimetrie mittels eines Spektrometers unterzogen wird;

   dadurch gekennzeichnet, daß die pH-Messung drei Messungen der Lichtabsorptionsfähigkeit A der Lösung umfaßt, von denen

   - die erste Messung mit einer Wellenlänge ($\lambda_M$) durchgeführt wird, die für eine maximale Absorptionsfähigkeit der basischen Form des Farbstoffs charakteristisch ist;

- die zweite Messung mit einer Wellenlänge ($\lambda_B$) durchgeführt wird, bei der der Farbstoff nicht absorbiert; und

- die dritte Messung mit einer um den isosbestischen Punkt des Farbstoffs liegenden Wellenlänge ($\lambda_I$) durchgeführt wird, um die begrenzte Stabilität des Farbstoffs über der Zeit zu berücksichtigen;

und eine Eichung durchgeführt wird, um einen Koeffizienten $A_M$, der charakteristisch für die Meßzelle (3) und den verwendeten Farbstoff ist, und einen Koeffizienten $K_M$ zu bestimmen, der die Emissions- und Detektionseigenschaften des Spektrometers berücksichtigt; und daß von den Ergebnissen der drei Messungen der den $CO_2$-Gehalt repräsentierende pH-Wert gemäß der Gleichung $P_H = P_K + \log A/(A_M-A)$ subtrahiert wird, wobei $P_K$ eine charakteristische Konstante des in der Meßzelle (3) enthaltenen Farbindikators ist und die Absorptionsfähigkeit A gemäß der Gleichung $A = \log I_{eB}/I_{eM} + K_M$ errechnet wird, wobei $I_{eB}$ die an dem Ausgang der Meßzelle (3) bei den entsprechenden Wellenlängen ($\lambda_B,\lambda_M$) gemessenen Lichtintensitäten bezeichnet.

2. Verfahren nach Anspruch 1, dadurch gekennzeichnet, daß der Farbindikator Thymolblau ist, die erste Messung mit einer Wellenlänge ($\lambda_I$) von ungefähr 596 Nanometer durchgeführt wird, die zweite Messung mit einer Wellenlänge ($\lambda_I$) von ungefähr 493 Nanometer durchgeführt wird und die dritte Messung mit einer Wellenlänge ($\lambda_B$) von über 700 Nanometer durchgeführt wird.

3. Vorrichtung zur Messung des Gehaltes von in Meerwasser aufgelöstem $CO_2$, mit einem Spektrometer, das mit einer Ausgabequelle für Meß-Wellenlängen und mit einem Empfänger (4) versehen ist, wobei die Vorrichtung ferner mit einer eine Farbindikatorlösung enthaltenden Meßzelle (3) versehen ist, die zum Eintauchen in das Meerwasser vorgesehen ist und eine Struktur aufweist, die ihr ermöglicht, ihre $CO_2$-Konzentration derjenigen des Meerwassers anzugleichen, mit Vorrichtungen (8,9), um den Lichtstrahl der Ausgabequelle (7) zu einem Lichteingang der Zelle (3) und anschließend von einem Lichtausgang der Zelle (3) zu dem Empfänger (4) leiten, dadurch gekennzeichnet, daß die Vorrichtung ferner Einrichtungen (26,27a,27b,27c) aufweist, um den aus der Zelle (3) austretenden optischen Strahl nacheinander mit unterschiedlichen Wellenlängen zu filtern, die zum Durchführen der Messungen nach dem Verfahren gemäß Anspruch 1 oder 2 erforderlich sind.

4. Vorrichtung nach Anspruch 3, dadurch gekennzeichnet, daß das Spektrometer einen optischen Separator, der zwischen der Meßzelle (3) und der Eingangs-Optik der Meßzelle (3) angeordnet ist, und einen Referenz-Lichtempfänger und eine Referenzweg-Linse aufweist, wobei der optische Separator einen Teil des von der Quelle ausgegebenen Lichtflusses wegnimmt und die Referenzweg-Linse diesen auf einen Referenz-Lichtempfänger lenkt, wobei der von der Vorrichtung gelieferte Meßwert aus einem Vergleich zwischen dem von dem Referenz-Lichtempfänger ausgegebenen Signal und dem von dem Empfänger ausgegebenem Signal resultiert.

5. Vorrichtung nach Anspruch 3 oder 4, dadurch gekennzeichnet, daß die Zelle (3) eine Aufnahme (18) zum Aufnehmen einer Farbindikatorlösung aufweist, wobei die Aufnahme an mindestens einer ihrer Wände durch eine für das $CO_2$ semipermeabele Membran (6) geschlossen ist.

6. Vorrichtung nach Anspruch 5, dadurch gekennzeichnet, daß die Vertiefung (18) der Zelle (3) eine rechteckige Nut ist, die durch das Gehäuse (17) verläuft und an vieren ihrer Seiten durch das das Gehäuse bildende Material und an zwei anderen ihrer Seiten durch semipermeabele Membranen (6) geschlossen ist.

7. Vorrichtung nach Anspruch 6, dadurch gekennzeichnet, daß die Membranen (6) an dem Gehäuse (17) mittels Halteteilen (23) gehalten sind, die an Verbindungsteilen (22) gehalten sind, welche zwischen den Halteteilen (23) und den Membranen (6) angeordnet sind.

8. Vorrichtung nach Anspruch 6 oder 7, dadurch gekennzeichnet, daß die Vorrichtungen zum Übertragen der Lichtimpulse optische Filter (8,8) sind, deren Enden in der Nut (18) ausmünden, und zwar auf der Ebene der Längsränder der Nut.

9. Vorrichtung nach Anspruch 8, dadurch gekennzeichnet, daß sie drei Interferenzfilter (27a,27b, 27c) aufweist, die an einer Drehscheibe (26) angeordnet sind, welche zwischen dem Ende der optischen Faser (9), das die aus der Zelle (3) austretenden Lichtimpulse überträgt, und dem Detektor (4) des Spektrometers angeordnet ist, wobei die drei Filter (27a,27b,27c) im Verlauf der Drehung der Drehscheibe (26) rechtwinklig an dem Ende der Faser (9) und dem dieser gegenüberliegenden Detektor (4) vorbeilaufen.

10. Vorrichtung nach einem der Ansprüche 3 bis 9, da-

durch gekennzeichnet, daß die Lichtleitvorichtungen (8,9) optische Fasern sind.

## Claims

1. A method for measuring the content of $CO_2$ dissolved in a sea-water, in which :

   - a measurement cell (3) is immersed in the sea-water, this measurement cell (3) containing a color indicator solution on which the pH measurements can be taken by colorimetry, and whose $CO_2$ concentration is equalized to that of the sea-water,
   - a pH measurement is taken on this solution by colorimetry by means of a spectrometer;

   characterized in that the pH measurement comprises three measurements of the solution optical absorbence A in which :

   - the first is made at a characteristic wavelength ($\lambda_M$) of an absorption peak of the basic form of the dye,
   - the second is made at a wavelength ($\lambda_B$) at which the dye does not absorb; and
   - the third is made at a wavelength ($\lambda_I$) near the isobestic point of the dye in order to take into account the limited stability of the dye in the time;

   as well as a sampling to determine a characteristic coefficient $A_M$ of the measurement cell (3) and the used dye and a coefficient $K_M$ taking into account the emission and detection characteristics of the spectrometer; and
   in that the pH value is deduced from the results of said three measurements corresponding to the $CO_2$ content according to the relation $P_H = P_K + \log A/(A_M-A)$, $P_K$ being a characteristic constant of the color indicator provided in the measurement cell (3) and absorbence A being obtained by the relation $A = \log Ie_B/Ie_M + K_M$, $Ie_B$ being the light intensities measured at the output of the measurement cell (3) at the corresponding wavelengths ($\lambda_B$, $\lambda_M$).

2. The method of Claim 1, characterized in that the color indicator is thymol blue, the first measurement being taken at a wavelength ($\lambda_I$) of about 596 nm, the second measurement being taken at a wavelength ($\lambda_I$) of about 493 nm and the third measurement being taken at a wavelength ($\lambda_B$) beyond 700 nm.

3. A device for measuring the content of $CO_2$ dissolved in sea-water comprising a spectrometer provided with a source (7) that emits in particular at the measurement wavelengths, and a receiver (4), said device also comprising a measurement cell (3) containing the color indicator solution, said measurement cell (3) being designed to be immersed in the sea-water and having a structure that enables it to equalize its dissolved $CO_2$ concentration to that of the sea-water, means (8, 9) to conduct the light beam from the emitting source (7) to an optical input of said cell (3), and then from an optical output of said cell (3) to the receiver (4), characterized in that it is provided, moreover, with means (26, 27a, 27b, 27c) to filter successively the optical beam at the output of the cell (3) at the different wavelengths required to take the measurements according to the method of Claims 1 or 2.

4. The device according to Claim 3, characterized in that the spectrometer comprises an optical separator located between the measurement cell (3) and the input optics of the measurement cell (3) and a reference photoreceiver and a reference channel lens, the optical separator sampling a part of the flux emitted by the source (7) and the reference channel lens sending it to the reference photoreceiver, the measurement supplied by the device resulting from the comparison between the signal supplied by the reference photoreceiver and the signal supplied by the receiver.

5. The device according to one of Claim 3 or 4, characterized in that the cell (3) comprises a housing (18) receiving a color indicator solution, this housing being closed on a least one of its walls by a membrane (6) that is semi-permeable to dissolved $CO_2$.

6. The device according to Claim 5, characterized in that the recess (18) of the cell (3) is a rectangular groove passing through the case (17) of the cell and closed on four of its sides by the material making up said case (17) and on another two of its sides by semi-permeable membranes (6).

7. The device according to Claim 6, characterized in that these membranes (6) are maintained on said case (17) by means of holding members (23) bearing on seals (22) inserted between said holding members (23) and said membranes (6).

8. The device according to any one of Claims 6 or 7, characterized in that the means for conveying the light pulse are optical fibers (8, 9) whose ends terminate in said groove (18) at the edges of the longitudinal ends of said groove.

9. The device according to Claim 8, characterized in that it is provided with three interference filters (27a, 27b, 27c) located on a rotating plate (26) inserted between on one hand the end of the optical fiber (9) conveying the light pulse at the output of the cell (3), and on the other hand the detector (4) of the spectrometer, said three filters (27a, 27b, 27c) passing successively during the rotation of said rotating plate (26) opposite the end of said fiber (9) and opposite the detector (4) facing it.

10. The device according to any one of Claims 3 to 9, characterized in that the optical conducting means (8, 9) are optical fibers.

FIG.1

FIG. 2

FIG.3

FIG.5

FIG. 4

FIG. 6